# EUROPEAN PATENT APPLICATION

(11) **EP 4 751 764 A1**
(43) Date of publication of application: **03.06.2026**
(21) Application number: 25218967.5
(22) Date of filing: 27.11.2025
(51) Int. Cl.: A61N 1/36, A61B 5/00, A61N 1/365

(54) **IMPLANTABLE DEVICE-BASED PERIPHERAL EDEMA STATUS MONITORING**

(30) Priority: 27.11.2024 US 202463725759 P; 25.11.2025 US 202519400433
(71) Applicant: Medtronic, Inc., Minneapolis, Minnesota 55432 (US)
(72) Inventor: O'Brien, Richard J., Minneapolis, 55432 (US); Zielinski, Todd M., Minneapolis, 55432 (US); Vaddiraju, Santhisagar, Minneapolis, 55432 (US); Ramos, Veronica, Minneapolis, 55432 (US); Gordon, Charles R., Minneapolis, 55432 (US); Peichel, David J., Minneapolis, 55432 (US); Zhao, Yanzhu, Minneapolis, 55432 (US); Cassens, Taya H., Minneapolis, 55432 (US); Opoku, Kwaku N., Minneapolis, 55432 (US); Slopsema, Julia P., Minneapolis, 55432 (US); Brink, Thaddeus S., Minneapolis, 55432 (US); Eggen, Michael D., Minneapolis, 55432 (US)
(74) Representative: Zimmermann & Partner Patentanwälte mbB

(57) **Abstract**

An example system includes an implantable medical device (IMD) that includes a housing configured for subcutaneous implantation proximate a lymphatic element of a patient; and a plurality of electrodes configured to sense an impedance signal indicative of one or more physiological parameters of the lymphatic element; and processing circuitry configured to: determine the one or more physiological parameters of the lymphatic element based on the impedance signal; determine a peripheral edema status of the patient based on the one or more physiological parameters; and output an indication of the peripheral edema status.

## Description

### FIELD

This application claims the benefit of U.S. Provisional Patent Application Serial No. 63/725,759, filed November 27, 2024, the entire content of which is incorporated herein by reference.

### FIELD

The disclosure relates generally to medical systems and, more particularly but not exclusively, medical systems configured to monitor a health condition status.

### BACKGROUND

Medical systems may monitor various types of data of a patient or a group of patients for one or several purposes. Amongst the numerous examples, some medical systems may record measurements of a patient, such as electrical activity of their heart and blood pressure, as indicia of physiologic health for that patient, which may be memorialized in raw data and/or processed data formats. Medical professionals may use such a medical system on their patients for a number of reasons, such as having the medical system record patient data for future use, e.g., in directing therapy for the patient. In some examples, the medical system may include one or more medical devices to collect various measurements used to detect changes in patient physiologic health.

### SUMMARY

An estimated 20% of Americans 51 years and older suffer from peripheral edema. In some examples, peripheral edema may result from deficiencies in venous or lymphatic systems (e.g., primary lymphedema), congestive heart failure (CHF), and certain medications. Peripheral edema, due to inadequate drainage of lymph fluid into the venous blood, may not be immediately evident, and a patient's weight may increase by substantial amount due to peripheral edema, such as nearly 10% before the peripheral edema is identified, and treatment called for. However, treatment for peripheral edema at such a stage has been mostly ineffective.

The present disclosure describes a medical system including one or more IMDs configured for subcutaneous implantation proximate a respective lymphatic element of a patient and configured to respectively sense impedance, temperature, and/or optical signals indicative of one or more physiological parameters of the respective lymphatic elements. The medical system, in accordance with the present disclosure, includes processing circuitry configured to determine the one or more physiological parameters of the respective lymphatic element(s) based on the respective impedance, temperature, and/or optical signal(s), determine a peripheral edema status of the patient based on the one or more physiological parameters, and output an indication of the peripheral edema status. The system of the present disclosure having one or more IMDs configured for subcutaneous implantation proximate a lymphatic element of a patient and configured to sense impedance and/or optical signal(s) indicative of one or more physiological parameters of lymphatic element(s) may help the system determine an occurrence/status of a peripheral edema earlier than previous methods that have been used to identify a peripheral edema in a patient, such as using patient weight gain to determine peripheral edema. The system of the present disclosure may identify a peripheral edema in a patient while the peripheral edema is more receptive to treatment, which may lead to improved outcomes of peripheral edema treatment.

For example, the system of the present disclosure may determine an occurrence/status of peripheral edema based on the impedance, temperature, and/or optical signal before symptoms occur and/or before such large volumes of fluid accumulate in a patient. In some examples, the system of the present disclosure being able to determine a patient is suffering a peripheral status or change thereto sooner may enable effective treatment to be administered to the patient to treat the peripheral edema before the peripheral edema becomes large enough that it becomes difficult to treat. In some examples, peripheral edema treatment may include one or more of medications (e.g., diuretics), compression garments, and/or stimulation therapy. In some examples, effectively treating a peripheral edema in a patient may help treat CHF in the patient. In some examples, the system of the present disclosure may determine a peripheral edema status of the patient while treatment to reduce the peripheral edema or cause thereof is being administered to determine whether the treatment being administered is effective and/or whether the treatment being administered may need to be adjusted. In some examples, the system of the present disclosure may additionally provide stimulation therapy in response to a determination of an occurrence/status of peripheral edema in a patient.

In one example, this disclosure describes a system comprising: an implantable medical device (IMD) comprising: a housing configured for subcutaneous implantation proximate a lymphatic element of a patient; and a plurality of electrodes configured to sense an impedance signal indicative of one or more physiological parameters of the lymphatic element; and processing circuitry configured to: determine the one or more physiological parameters of the lymphatic element based on the impedance signal; determine a peripheral edema status of the patient based on the one or more physiological parameters; and output an indication of the peripheral edema status.

In another example, this disclosure describes a method of determining a peripheral edema status of a patient, the method comprising: sensing, via an implantable medical device (IMD) configured for subcutaneous implantation proximate a lymphatic element of a patient, an impedance signal indicative of one or more physiological parameters of the lymphatic element; determining, via processing circuitry, the one or more physiological parameters of the lymphatic element based on the impedance signal; determining, via the processing circuitry, a peripheral edema status of the patient based on the one or more physiological parameters; and outputting, via the processing circuitry, an indication of the peripheral edema status.

In another example, this disclosure describes a computer readable medium storing instructions that when executed by one or more processors cause the one or more processors to perform sensing, via an implantable medical device (IMD) configured for subcutaneous implantation proximate a lymphatic element of a patient, an impedance signal indicative of one or more physiological parameters of the lymphatic element; determining the one or more physiological parameters of the lymphatic element based on the impedance signal; determining a peripheral edema status of the patient based on the one or more physiological parameters; and outputting an indication of the peripheral edema status.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF THE DRAWINGS

The details of one or more examples of this disclosure are set forth in the accompanying drawings and the description below. Other features, objects, and advantages of this disclosure will be apparent from the description and drawings, and from the claims.
FIG. 1 is a conceptual drawing illustrating an example system configured to determine a peripheral edema status of a patient in accordance with one or more techniques of this disclosure
FIG. 2 is a block diagram illustrating an example system configured to detect a peripheral edema status of a patient in accordance with one or more techniques of this disclosure.
FIG. 3 is a conceptual drawing illustrating an example implantable medical device in accordance with one or more techniques of this disclosure.
FIG. 4A is a block diagram illustrating an example lymphatic focused implantable medical device in accordance with one or more techniques of this disclosure.
FIG. 4B is a block diagram illustrating an example general implantable medical device in accordance with one or more techniques of this disclosure.
FIG. 5 is a block diagram illustrating an example computing device in accordance with one or more techniques described herein.
FIG. 6 is a flowchart illustrating an example method of determining a peripheral edema status of a patient.

Like reference characters denote like elements throughout the description and figures.

### DETAILED DESCRIPTION

A variety of types of implantable and external devices are configured to detect a status of health conditions based on sensed physiological parameters. During heart failure (HF) episodes (e.g., diastolic heart failure or systolic heart failure), such as acute decompensated heart failure (ADHF), kidney flow and function are overactivated, which may lead to less diuresis and a higher blood volume, or volume overload. Further, an already weakened heart may struggle to effectively process an increased blood volume, which may lead to congestion and fluid movement into nearby organs, potentially causing dyspnea and edema of one or more organs. Complications from ADHF may include, for example, hemodynamic failure, arrhythmias (e.g., atrial fibrillation, ventricular tachycardia, and ventricular fibrillation), organ damage due to oxygen shortage (e.g., liver, kidney, and brain), acute respiratory distress, cardiac valve dysfunction, leg venous stasis, and ulcers.

However, peripheral edema may not be immediately evident, and a patient's weight may increase by nearly 10% before peripheral edema is identified, and treatment called for, but attempting to effectively treat peripheral edema after peripheral edema symptoms occur and/or such large volumes of fluid have accumulated in patient has been mostly ineffective.

According to some example techniques described herein, a medical system including one or more IMDs configured for subcutaneous implantation proximate a lymphatic element of a patient and configured to sense an impedance and/or optical signal indicative of one or more physiological parameters of the lymphatic element may determine an occurrence of peripheral edema based on the impedance and/or optical signal before symptoms occur and/or well before such large volumes of fluid (e.g., an increase of nearly 10% weight gain) accumulate in a patient. In some examples, the one or more IMDs configured for subcutaneous implantation proximate a lymphatic element may be referred to as "lymphatic focused IMDs". Although referred to as lymphatic focused IMDs, IMDs configured for subcutaneous implantation proximate a lymphatic element may sense one or more physiological parameters not particularly related to the lymphatic system in addition to one or more lymphatic parameters. In some examples, by determining a patient is suffering a peripheral status or change thereto sooner improves the determination of a peripheral edema status of a patient which may enable effective treatment to be administered to the patient to treat the peripheral edema before the peripheral edema becomes large enough that it becomes difficult to treat. In some examples, peripheral edema treatment may include one or more of medications (e.g., diuretics) and/or compression garments. In some examples, effectively treating a peripheral edema in a patient may help treat CHF in the patient.

In some examples, systems according to this disclosure may include one or more lymphatic focused IMDs that are configured for subcutaneous implantation proximate a lymphatic element of a patient via injection. In some examples, additionally or alternatively, one or more of lymphatic focused IMDs may be configured for subcutaneous implantation proximate a lymphatic element of a patient via a surgical procedure. In some examples, one or more of lymphatic focused IMDs may respectively include a plurality of electrodes configured to sense an impedance and/or optical signal indicative of one or more physiological parameters of the lymphatic element. In some examples, the impedance signal may include at least one of real impedance and/or reactive impedance.

In some examples, processing circuitry of the system may include one or more of processing circuitry of the lymphatic focused IMDs, processing circuitry of an external computing device, such as a smartphone, smart watch, computer, or server, and/or processing circuitry of an IMD that is configured to sense a variety of health condition statuses, such as an implantable cardiac monitor (ICM). In some examples, an IMD that is configured to sense a variety of physiological parameters and/or health condition statuses may be referred to as a "general IMD." Although referred to as a general IMD herein, such an IMD may be configured to sense one or more parameters of a lymphatic element in some examples.

In some examples, lymphatic focused IMDs having a plurality of electrodes being configured to sense impedance signal indicative of one or more physiological parameters of the lymphatic element and/or including an optical sensor configured to sense optical signals indicative of one or more physiological parameters of the lymphatic element may enable the lymphatic focused IMDs to be smaller in size than general IMDs and/or use reduced amounts of power in comparison to a general IMD. In some examples, lymphatic focused IMDs being smaller in size (e.g., between 5 millimeters (mm) and 30 mm in length, between 2 mm and 4 mm in diameter, and less than 1 cubic centimeter (cm) in volume) than general IMDs (e.g., may enable lymphatic focused IMDs to be more easily implanted proximate a particular lymphatic element in a patient, such as in a limb of patient.

In some examples, processing circuitry of the system may be configured to determine one or more physiological parameters of the respective lymphatic element based on the impedance, temperature, and/or optical signal(s) respectively sensed by the plurality of electrodes, temperature sensors, or optical sensor(s) of the one or more lymphatic focused IMDs. In some examples, the physiological parameters may include at least one of afferent lymph flow, efferent lymph flow, lymph node volume, lymph node geometric size, lymph node arterial waveform, lymph node arterial pressure, lymph node activity of the respective lymphatic element. In some examples, the physiological parameters may additionally or alternatively include inflammatory response and/or cytokine release. Table 1 shows some non-limiting examples of signals/measurements respectively sensed/measured by IMDs 102 and/or IMD 104 that may be used to determine one or more physiological parameters.

**Table 1. Physiologic parameters and sensed signals.**

| **Physiologic Change Parameter** | **Sensed Signal(s)** |
|---|---|
| Afferent and efferent lymph flow | Real Impedance (R) |
| Node global volume status | Real (R) and Reactive Impedance (X_{c}) |
| Node geometric size | Real (R) and Reactive Impedance (X_{c}) |
| Node arterial waveform anomalies | Optical Signal and/or Real Impedance (R) |
| Node arterial pressure | Optical Signal and/or Real Impedance (R) |
| Node lymph activity | Real (R) and Reactive Impedance (X_{c}) |
| Interstitial fluid amount | Real Impedance (R) |
| Local and global inflammatory response | Real Impedance (R) and Temperature |
| Lymphatic Vessel Contractility | Optical Signal and/or Real Impedance (R) and/or Reactive Impedance (X_{c}) |
| Lymphatic Vessel Contractile Volume Transfer (Localized) | Real Impedance (R) |
| Central Venous Volume Change | Real Impedance (R) |

In some examples, processing circuitry of the system may be configured to determine a peripheral edema status of the patient based on the one or more physiological parameters. In some examples, a peripheral edema status may include one or more of an indication of whether a patient is suffering from a peripheral edema, an indication of whether a peripheral edema is changing, such as worsening or improving, over a period of time, whether a peripheral edema indicates treatment and/or a change in treatment be administered. In some examples, processing circuitry of the system may be configured to output an indication of the peripheral edema status to a computing device that may facilitate determinations of patient wellness, determination of severity of peripheral edema, and/or determination of risk of suffering severe peripheral edema (e.g., such as 10% or more weight gain) in the near future, such as within the next 7 days, within the next 14 days, within the next month, etc., and may lead to clinical interventions to suppress peripheral edema such as medications and/or compression garments.

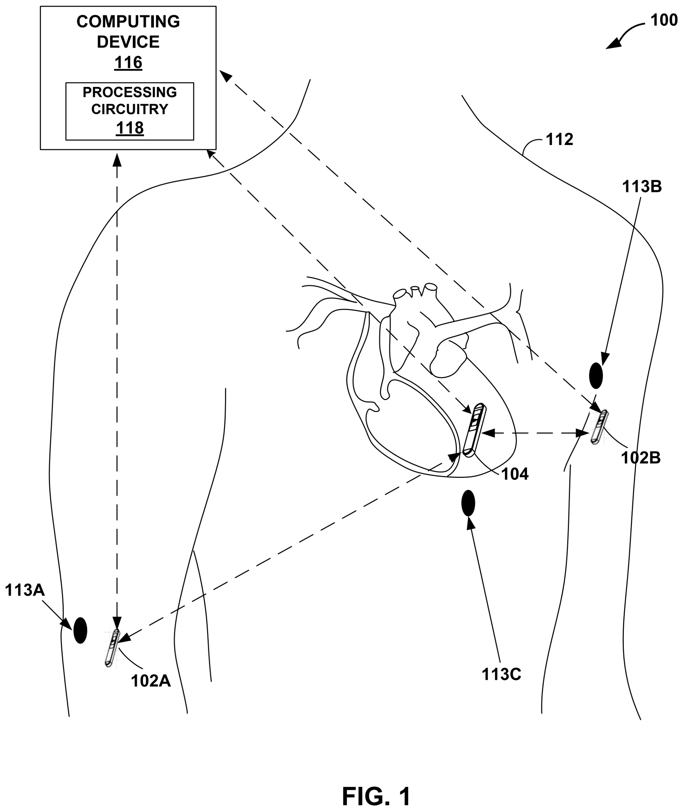

FIG. 1 is a conceptual drawing illustrating an example of system 100 configured to determine a peripheral edema status of patient 112. In some examples, system 100 may include one or more IMDs 102A, 102B (collectively referred to as "IMD(s) 102") configured for subcutaneous implantation proximate a respective lymphatic element of a patient 112. In some examples, IMDs 102 may be referred to as "lymphatic focused IMD(s) 102." In some examples, a lymphatic element may include a lymph node and/or lymph vessel of the patient 112. In some examples, IMD(s) 102 may be implanted in a limb of patient 112, such as an arm or leg of patient 112. In some examples, IMD(s) 102 may be implanted in positions of patient that include a higher concentration of lymphatic elements, such as near the arm pit, elbow, groin, knee, wrist, and/or ankle area. In some examples, IMD(s) 102 may additionally or alternatively be implanted in the torso, abdomen, clavicle, and/or neck area. Although multiple implant locations are shown for, IMD(s) 102 may be implanted anywhere on or within patient 112 based on a clinician's desire to record any data from that location. In some examples, IMD 104 may be a configured to monitor a variety of physiological parameters, such as blood pressure, electrocardiogram (ECG) signals, heart rate, cardiac output, heart sounds, impedance, cardiac motion, respiration signals, perfusion signals, activity and/or posture signals, pressure signals, blood oxygen saturation signals, body composition, fluid impedance signals, and blood glucose or other blood constituent signals. In some examples, IMD 104 may be configured to sense an impedance signal and/or optical signal indicative of one or more physiological parameters of a respective lymphatic element, such as 113C. In some examples, system 100 may include one or more IMD(s) 102 and IMD 104. In some examples, IMD 104 may be referred to as "general IMD 104." In some examples, IMD 102 may sense additional physiological signals indicative of additional physiological parameters in addition to sensing an impedance signal and/or optical signal indicative one or more physiological parameters of a respective lymphatic element.

In some examples, system 100 may include computing device 116. In some examples, computing device 116 may include processing circuitry 118 and communication circuitry configured to communicate with other electronic devices, such as IMD(s) 102, IMD 104, or other external computing devices. In some examples, system 100 may include one or more IMD(s) 102 but not include IMD 104. In some examples, system 100 may include one or more IMD(s) 102 and one or more of computing device 116 and/or IMD 104.

Although the techniques for determining a peripheral edema status of patient 112 are described herein primarily (e.g., with respect to FIGS. 1-5) as being performed by processing circuitry 118 of computing device 116, such techniques may be performed, in whole or part, by processing circuitry of any one or more devices of system 100, such as processing circuitry 50A of IMD 104, processing circuitry 50 of IMD 102, processing circuitry 118 of computing device 116, and/or processing circuitry of computing systems 20. In some examples, determining a peripheral edema status of patient 112 may be performed by one or more of processing circuitry 118 and/or processing circuitry 50A of IMD 104.

IMD(s) 102 may be implanted or inserted anywhere onto or within patient 112, in accordance with one or more techniques of this disclosure. In some examples, IMD(s) 102 may be implanted with a medical tool to inject one or more IMD(s) 102 subcutaneously within patient 112, such as subcutaneously within a limb of patient 112. In some examples, one or more of IMD(s) 102 may respectively include a housing configured for subcutaneous implantation proximate a respective lymphatic element 113A, 113B, 113C (collectively referred to as "lymphatic element 113") of patient 112 and a plurality of electrodes configured to sense an impedance signal indicative of one or more physiological parameters of the respective lymphatic element 113. For example, IMD 102A may be configured proximate to lymphatic element 113A and configured to sense an impedance signal indicative of one or more physiological parameters of the respective lymphatic element 113A and IMD 102B may be configured proximate to lymphatic element 113B and configured to sense an impedance signal indicative of one or more physiological parameters of the respective lymphatic element 113B.

In some examples, one or more of IMD(s) 102 may respectively include a temperature sensor configured to sense temperature indicative of one or more physiological parameters of the respective lymphatic element 113. For example, IMD 102A may be configured proximate to lymphatic element 113A and configured to sense first temperature(s) indicative of one or more physiological parameters of the respective lymphatic element 113A and IMD 102B may be configured proximate to lymphatic element 113B and configured to sense second temperature(s) indicative of one or more physiological parameters of the respective lymphatic element 113B. In some examples, processing circuitry 118 may determine at least one of a first inflammatory response status of patient 112 based on the first temperature(s) and/or determine at least one of second inflammatory response status of patient 112 based on the second temperature(s).

In some examples, processing circuitry 118 may determine a peripheral edema status based on the one or more first physiological parameters of the respective lymphatic element 113A and the one or more second physiological parameters of the respective lymphatic element 113B.

In some examples, IMD 102A may additionally include an optical sensor configured to sense first optical signal(s). In some examples, IMD 102B may additionally include a second optical sensor configured to sense second optical signal(s). In some examples, processing circuitry 118 may determine at least one of first lymph node arterial waveform or first lymph node arterial pressure of patient 112 based on the first optical signal(s) and/or determine at least one of second lymph node arterial waveform or second lymph node arterial pressure of patient 112 based on the second optical signal(s).

In some examples, first physiological parameters include at least one of afferent lymph flow, efferent lymph flow, lymph node volume, lymph node geometric size, lymph node arterial waveform, lymph node arterial pressure, and/or lymph node activity of the first lymphatic element. In some examples, second physiological parameters include at least one of afferent lymph flow, efferent lymph flow, lymph node volume, lymph node geometric size, lymph node arterial waveform, lymph node arterial pressure, and/or lymph node activity of the second lymphatic element. In some examples, processing circuitry 118 may determine a peripheral edema status based on the one or more first physiological parameters, the one or more second physiological parameters, the at least one of first lymph node arterial waveform or first lymph node arterial pressure, and the at least one of second lymph node arterial waveform or second lymph node arterial pressure. In some examples, processing circuitry 118 may determine a peripheral edema status based on the one or more first physiological parameters, the one or more second physiological parameters, the fist inflammatory response status and the second inflammatory response status.

The location of the IMDs 102 and lymphatic elements 113 shown in FIG. 1 are examples, but one or more IMDs 102 and/or one or more lymphatic elements 113 may be positioned in a variety of other locations, such as near the arm pit, elbow, groin, knee, and/or ankle area. In some examples, locations of IMD(s) 102 and/or one or more lymphatic elements 113 may additionally or alternatively include the torso, abdomen, clavicle, and/or neck area. In some examples, the impedance signal may include at least one of real impedance and/or reactive impedance.

In some examples, processing circuitry 118 may be configured to determine one or more physiological parameters of the respective lymphatic element 113 based on the impedance signal(s) respectively sensed by the plurality of electrodes of the one or more lymphatic focused IMDs 102. In some examples, the physiological parameters may include at least one of afferent lymph flow, efferent lymph flow, lymph node volume, lymph node geometric size, lymph node arterial waveform, lymph node arterial pressure, lymph node activity of the respective lymphatic element 113. In some examples, the physiological parameters may additionally or alternatively include inflammatory response and/or cytokine release. In some examples, processing circuitry 118 may determine a value or level of inflammatory response based on temperatures sensed by the respective temperature sensors of the respective one or more IMDs 102.

In some examples, processing circuitry 118 may be configured to determine a peripheral edema status of the patient based on the one or more physiological parameters. In some examples, a peripheral edema status may include one or more of an indication of whether a patient is suffering from a peripheral edema, an indication of whether a peripheral edema is changing, such as worsening or improving, over a period of time, whether a peripheral edema indicates treatment and/or a change in treatment be administered. In some examples, processing circuitry 118 may be configured to output an indication of the peripheral edema status, such as to computing device 116 (e.g., such as when processing circuitry 50A determines a peripheral edema status of the patient), clinician computing devices 38, and/or computing system 20, that may facilitate determinations of patient wellness, determination of severity of peripheral edema, and/or determination of risk of suffering severe peripheral edema (e.g., such as 10% or more weight gain) in the near future, such as within the next 7 days, within the next 14 days, within the next month, etc., and may lead to clinical interventions to suppress peripheral edema such as medications and/or compression garments. In some examples, processing circuitry 118 may be configured to output one or more of tactile, audio, and/or visual output of an indication of the peripheral edema status.

In some examples, processing circuitry 118 may determine a peripheral edema status of patient 112 while treatment to reduce the peripheral edema or cause thereof is being administered to patient 112 and determine whether the treatment being administered to patient 112 is effective and/or whether the treatment being administered to patient 112 should be adjusted in response to a determination that the current treatment is ineffective. In some examples, peripheral edema treatment may include one or more of medications (e.g., diuretics), compression garments, and/or stimulation therapy. In some examples, effectively treating a peripheral edema in a patient may help treat CHF in the patient.

In some examples, processing circuitry 118 may determine a location of a peripheral edema of patient 112 based on the one or more physiological parameters that are respectively based on one or more sensed signals, such as impedance, optical signal, and/or temperature, sensed via one or more IMDs 102. In some examples, processing circuitry 118 may determine a particular treatment to administer to patient, such as type and/or amount of treatment, based on the determined location of the peripheral edema. In some examples, processing circuitry 118 may determine one or more particular IMDs 102 to provide stimulation treatment in response to the determined location of the peripheral edema. For example, processing circuitry 118 may determine to have one or more IMDs 102 that are within a particular distance threshold of the determined location of the peripheral edema to provide stimulation treatment to treat the peripheral edema.

In some examples, processing circuitry 118 may determine a cause of a peripheral edema of patient 112 based on the one or more physiological parameters that are respectively based on one or more sensed signals, such as impedance, optical signal, and/or temperature, sensed via one or more IMDs 102. In some examples, processing circuitry 118 may determine whether a peripheral edema of patient 112 is lymphedema or HF caused edema based on the one or more physiological parameters that are respectively based on one or more sensed signals, such as impedance, optical signal, and/or temperature, sensed via one or more IMDs 102.

In some examples, system 100 may provide stimulation therapy, such as activating neural pathways using neurostimulation, to promote diuresis, such as in response to a determination of an occurrence/status of peripheral edema. In some examples, system 100 may utilize devices and/or techniques described in commonly-assigned U.S. Application No. 18/427,933 by Kornet et al., entitled "LATERAL EPIDURAL STIMULATION TO AFFECT KIDNEY FUNCTION," filed on January 31, 2024, which is incorporated herein by reference in its entirety, to promote diuresis by activating neural pathways using neurostimulation.

In some examples, system 100 may further include one or more devices, such as described in U.S. Application No. 18/427,933, configured to activate the renal nerves (e.g., afferent nerves) through electrical stimulation of the preganglionic dorsal root fibers of the corresponding dorsal roots of such renal nerves to mimic the electrical activation signals of such renal nerves to innervate at least one of the patient's kidneys to increase diuresis as part of the reno-renal reflex.

In some examples, system 100 may provide stellate ganglion stimulation therapy to increase blood flow and circulation in one or more limbs of patient 112, such as arm(s), and/or provide pain relief, such as in response to a determination of an occurrence/status of peripheral edema. In some examples, system 100 may determine to provide stellate ganglion stimulation therapy and/or muscle stimulation, such as limb muscle stimulation, in response to determining the edema was caused by lymphedema. In some examples, limb muscle stimulation may be provided via one or more IMDs 102. In some examples, system 100 may provide stellate ganglion nerve block therapy such as in response to a determination of an occurrence/status of peripheral edema.

In some examples, one or more of IMD(s) 102 and/or IMD 104 may be configured to provide electrostimulation, such as via one or more electrodes of a respective IMD. In some examples, one or more of IMD(s) 102 and/or IMD 104 may be configured to provide electrostimulation of muscles near a respective lymphatic element 113 to compress vessels and induct flow to promote diuresis.

In some examples, one or more of IMD(s) 102 and/or IMD 104 may be configured to provide electrostimulation based on a determination of a peripheral edema status of patient 112 and/or a determination of whether the treatment being administered to patient 112 is effective and/or whether the treatment being administered to patient 112 should be adjusted based on a determination that the current treatment is ineffective.

In some examples, processing circuitry 118 may be configured to output one or more of tactile, audio, and/or visual output of an indication of the peripheral edema status and/or an indication that treatment, such as electrostimulation or medication, should be applied to patient 112 in the near future, such as within 24 hours, 12 hours, 8 hours, etc. In some examples, patient 112 may provide input into computing device 116 of a particular time selected by patient 112 for one or more of IMD(s) 102 and/or IMD 104 to begin to provide electrostimulation therapy. In some examples, processing circuitry 118 may cause one or more of IMD(s) 102 and/or IMD 104 to begin to provide electrostimulation therapy based on the particular time selected by patient 112. Alternatively, in some embodiments a patient may select convenient time for the effects of therapy to be felt. For example, a patient may typically need to urinate an hour or two after beginning a therapy that promotes diuresis. In this example, the patient may select a time that will be convenient for urination (or other effect to be felt). In such embodiments, the therapy may then be scheduled to begin at the selected time minus a delay period known (e.g., generally or for the particular patient) to occur between the initiation of therapy and the effect felt by the patient. In some such embodiments, the progress of therapy or the effect felt by the patient may be monitored and adjusted in real time to help ensure that the effect is felt at the scheduled time (*e.g.,* if therapy is progressing faster than expected, the therapy may be slowed down; or if therapy is not progressing fast enough, the therapy may be accelerated). In some examples, processing circuitry 118 may store patient preference electrostimulation times to provide electrostimulation therapy and be configured to provide electrostimulation therapy based on a determination of a peripheral edema status and the stored patient preference electrostimulation times.

In some examples, one or more of IMD(s) 102 and/or IMD 104 may continue to monitor a peripheral edema status of patient 112 while electrostimulation therapy is provided and determine whether the electrostimulation therapy being administered to patient 112 is effective. In some examples, in response to a determination the electrostimulation therapy is effective in treating the peripheral edema, such as reducing fluid levels, processing circuitry 118 may determine to end the electrostimulation therapy.

In some embodiments, the electrostimulation device(s) may be actively powered, *e.g.,* by an onboard battery or other power source. In some embodiments, the electrostimulation device(s) may be passively powered by another device, such as a device external to the patient. In such embodiments, the patient may bring a power source device close to the electrostimulation device implant location at the time when therapy is to commence (*e.g.,* based on the indication for treatment or based on a reminder correlated to a previously scheduled treatment/patient effect time). The external power source device may be integrated into a patch, belt, or other apparatus configured to hold the power source device in place for an appropriate time to deliver to the electrostimulation device (e.g., through induction) the power that will be used for delivering therapy.

In some examples, computing device 116 may be in wireless communication with one or more of IMD(s) 102 and/or IMD 104. In some examples, computing device 116 may be a computing device used in a home, ambulatory, clinic, or hospital setting. Computing device 116 may include, for example, a desktop computer, a laptop computer, a workstation, a server, a mainframe, a cloud computing system, a smartphone, a smartwatch, combinations thereof, or the like. Computing device 116 may include a network interface card, such as an Ethernet card, an optical transceiver, a radio frequency transceiver, or any other type of device that can send and receive information. For example, computing device 116 may include a radio transceiver configured for communication according to standards or protocols, such as 3G, 4G, 5G, Wi-Fi (e.g., 802.11 or 802.15 ZigBee), Bluetooth^{®}, or Bluetooth^{®} Low Energy (BLE).

In some examples, IMD(s) 102 may be in wireless communication with IMD 104 and/or computing device 116 according to the Bluetooth^{®}, Bluetooth^{®} Low Energy (BLE), radio frequency (RF), or other wireless communication protocols, as examples. In some examples, one or more IMD(s) 102 may be in wireless communication with one or more other IMD(s) 102.

In some examples, IMD 104 takes the form of the LINQ^{™} ICM or LINQ II^{™} available from Medtronic, Inc., or an ICM based on thereon. IMD 104 may be implanted or secured to the skin of a patient and may be a wearable device similarly in wireless communication with one or more other devices not pictured in FIG. 1.

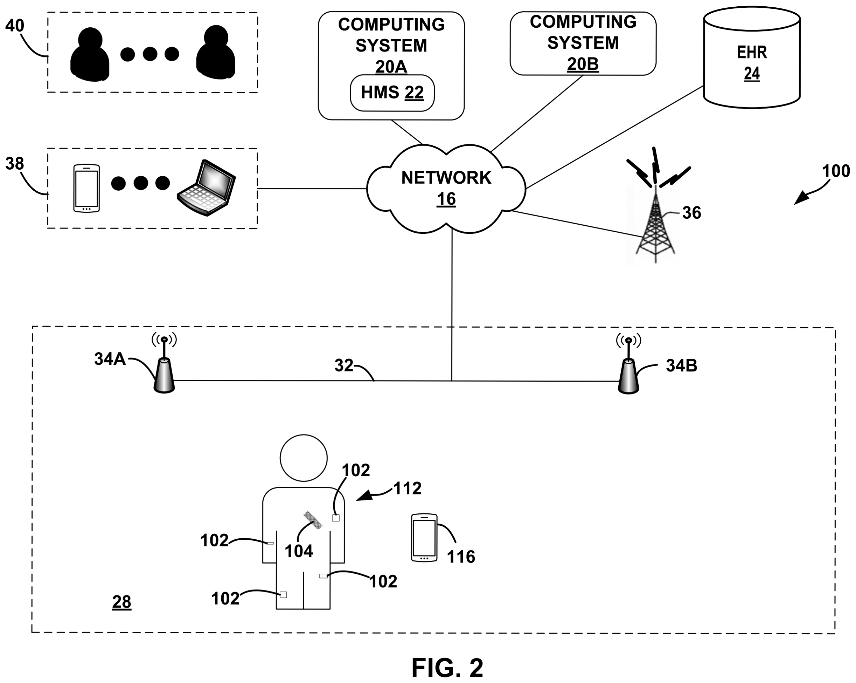

FIG. 2 is a block diagram illustrating an example system 100 configured determine a peripheral edema status of patient 112, and to respond to such detection, in accordance with one or more techniques of this disclosure. Patient 112 ordinarily, but not necessarily, will be a human. For example, patient 112 may be an animal needing ongoing monitoring for peripheral edema status. As used herein, the terms "detect," "detection," and the like may refer to detection of a status of a peripheral edema presently (at the time or the period of time the data is collected) being experienced by patient 112, as well as detection based on the data that the condition of patient 112 is such that they have a suprathreshold likelihood of experiencing the peripheral edema within a particular timeframe, e.g., prediction of the peripheral edema. The example techniques may be used with one or more patient sensing devices, e.g., IMDs 102 (collectively, "IMDs 102"), which may be in wireless communication with each other and/or may be in wireless communication with one or more patient computing devices, e.g., computing device 116 and/or IMD 104. Although not illustrated in FIG. 2, IMDs 102 may include electrodes and other sensors to sense physiological signals, such as impedance signals indicative of one or more physiological parameters of a respective lymphatic element and/or optical signals indicative of one or more physiological parameters of a respective lymphatic element, of patient 112, and may collect and store sensed physiological data based on the signals and detect a peripheral edema status based on the data. In some examples, IMDs 102 may include one or more electrodes to provide electrostimulation. In some examples, IMDs 102 may include one or more electrodes that sense physiological signals, such as impedance signals indicative of one or more physiological parameters of a respective lymphatic element, and provide electrostimulation.

In some examples, one or more IMDs 102 may be configured to transmit and/or receive communication signals via molecular communication and/or encoding stimulation pulses. In some examples, additionally or alternatively, one or more IMDs 102 may communicate with one or more other IMDs 102 via molecular communication and/or encoding stimulation pulses. For example, one or more IMDs 102 transmit communication signals via the nervous system of patient 112 to be received by one or more other IMDs 102. In some examples, in which one or more IMDs 102 may be configured to transmit and/or receive communication signals via molecular communication and/or encoding stimulation pulses, at least one of the one or more IMDs 102 may be configured for wireless communication with computing devices 116 and/or IMD 104.

Computing devices 116 and/or IMD 104 are configured for wireless communication with lymphatic focused IMDs 102. Computing devices 116 and/or general IMD 104 retrieve sensed physiological data from lymphatic focused IMDs 102 that was collected and stored by the lymphatic focused IMDs 102. In some examples, computing devices 116 take the form of personal computing devices of patient 112. For example, computing device 116 may take the form of a smartphone of patient 112 or a smartwatch or other smart apparel of patient 112. In some examples, computing devices 116 may be any computing device configured for wireless communication with IMDs 102 such as a desktop, laptop, or tablet computer. Computing devices 116 and/or IMD 104 may communicate with IMDs 102 and each other according to the Bluetooth^{®} or Bluetooth^{®} Low Energy (BLE) protocols, as examples

One or more of computing devices 116 and/or IMD 104 may be configured to communicate with a variety of other devices or systems via a network 16. For example, computing device 116 may be configured to communicate with one or more computing systems, e.g., computing systems 20A and 20B (collectively, "computing systems 20") via network 16. Computing systems 20A and 20B may be respectively managed by manufacturers of IMDs 102 and computing devices 116 to, for example, provide cloud storage and analysis of collected data, maintenance and software services, or other networked functionality for their respective devices and users thereof. Computing system 20A may comprise, or may be implemented by, the Medtronic Carelink^{™} Network, in some examples. In the example illustrated by FIG. 1, computing system 20A implements a health monitoring system (HMS) 22, although in other examples, either of both of computing systems 20 may implement HMS 22. As will be described in greater detail below, HMS 22 may facilitate detection of peripheral edema status of patient 112 by system 100, and the responses of system 100 to detection of peripheral edema status.

Computing device 116 may transmit data, including data retrieved from lymphatic focused IMDs 102 to computing system(s) 20 via network 16. The data may include sensed data, e.g., impedance signals indicative of one or more physiological parameters of the lymphatic element or values of impedance measured by lymphatic focused IMDs 102 and, in some cases computing device 116, data regarding peripheral edema status determined by lymphatic focused IMDs 102 and computing device 116, and other physiological signals or data recorded by IMDs 102, IMD 104, and/or computing device 116. HMS 22 may also retrieve data regarding patient 112 from one or more sources of electronic health records (EHR) 24 via network. EHR 24 may include data regarding historical (e.g., baseline) physiological parameter values, previous health events and treatments, disease states, comorbidities, demographics, height, weight, and body mass index (BMI), as examples, of patients including patient 112. HMS 22 may use data from EHR 24 to configure algorithms implemented by lymphatic focused IMDs 102, general IMD 104, and/or computing device 116 to determine a peripheral edema status for patient 112. In some examples, HMS 22 provides data from EHR 24 to computing device 116 and/or general IMD 104 for storage therein and use as part of their algorithms for determine a peripheral edema status. In some examples, physiological parameter values from EHR 24 and/or from IMD 104 may be used in combination with lymphatic parameters sensed via IMD 102 and/or peripheral edema status determined based on lymphatic parameters sensed via IMD 102 to determine a HF status (e.g., detect or predict worsening or improving HF) of patient 112.

Network 16 may include one or more computing devices, such as one or more non-edge switches, routers, hubs, gateways, security devices such as firewalls, intrusion detection, and/or intrusion prevention devices, servers, cellular base stations and nodes, wireless access points, bridges, cable modems, application accelerators, or other network devices. Network 16 may include one or more networks administered by service providers, and may thus form part of a large-scale public network infrastructure, e.g., the Internet. Network 16 may provide computing devices and systems, such as those illustrated in FIG. 2, access to the Internet, and may provide a communication framework that allows the computing devices and systems to communicate with one another. In some examples, network 16 may include a private network that provides a communication framework that allows the computing devices and systems illustrated in FIG. 2 to communicate with each other, but isolates some of the data flows from devices external to the private network for security purposes. In some examples, the communications between the computing devices and systems illustrated in FIG. 1 are encrypted.

One or more of lymphatic focused IMDs 102 and/or general IMD 104 may be configured to transmit data, such as sensed, measured, and/or determined values of physiological parameters (e.g., an impedance signal indicative of one or more physiological parameters of the lymphatic element, impedance measurements indicative of one or more physiological parameters of the lymphatic element, impedance scores indicative of one or more physiological parameters of the lymphatic element, one or more physiological parameters of the lymphatic element, optical signals indicative of one or more physiological parameters of the lymphatic element, values of afferent lymph flow, efferent lymph flow, lymph node volume, lymph node geometric size, lymph node arterial waveform, lymph node arterial pressure, lymph node activity, inflammatory response, and/or cytokine release, etc.), to wireless access points 34A, 34B (collectively, "wireless access points 34"), and/or computing device 116. Wireless access points 34 and/or computing device 116 may then communicate the retrieved data to computing systems 20 via network 16.

Wireless access points 34 may include a device that connects to network 16 via any of a variety of connections, such as telephone dial-up, digital subscriber line (DSL), or cable modem connections. In other examples, wireless access points 34 may be coupled to network 16 through different forms of connections, including wired or wireless connections. In some examples, IMDs 102 and/or IMD 104 may be configured to transmit data, such as one or more physiological parameters, to wireless access points 34. Wireless access points 34 may then communicate the retrieved data to computing systems 20 via network 16.

One or more of lymphatic focused IMDs 102 may transmit data over a wired or wireless connection to computing system 20, to computing device 116, or to general IMD 104. For example, computing device 116 may receive data from lymphatic focused IMDs 102 or from general IMD 104. In another example, computing device 116 may receive data from computing system 20 or from lymphatic focused IMDs 102, such as physiological parameter values, diagnostic states, or probability scores, via network 16. In such examples, computing device 116 may determine the data received from computing system 20 or from IMDs 102 and may store the data to a storage device in the computing device(s) accordingly.

In some cases, computing system 20 may be configured to provide a secure storage site for data that has been collected from IMDs 102 and/or IMD 104. In some instances, computing system 20 may include a database that stores medical- and health-related data. For example, computing system 20 may include a cloud server or other remote server that stores data collected from IMDs 102 and/or IMD 104. In some cases, computing system 20 may assemble data in web pages or other documents for viewing by trained professionals, such as clinicians, via clinician computing devices 38. One or more aspects of the example system described with reference to FIG. 2 may be implemented with general network technology and functionality, which may be similar to that provided by the Medtronic CareLink^{®} Network.

In some examples, one or more of clinician computing devices 38 may be a tablet or other smart device located with a clinician, by which the clinician may program, receive alerts from, and/or interrogate IMDs 102. For example, the clinician may access data collected by IMDs 102 through a clinician computing device 38, such as when patient 112 is in between clinician visits, to check on a status of a medical condition. In some examples, the clinician may enter instructions for a medical intervention for patient 112 into an application executed by clinician computing device 38, such as based on a status of a patient condition determined by IMDs 102, IMD 104, computing device 116, computing system 20, or any combination thereof, or based on other patient data known to the clinician.

One clinician computing device 38 may transmit instructions for medical intervention to another of clinician computing devices 38 located with patient 112, a caregiver of patient 112, and/or computing device 116, such as for self-administered therapies. For example, such instructions for medical intervention may include an instruction to change a drug dosage, timing, or selection, to schedule a visit with the clinician, or to seek medical attention. In further examples, a clinician computing device 38 may generate an alert to patient 112 (or relay an alert determined by IMDs 102, IMD 104, computing device 116, or computing system 20) based on a probability score (e.g., posterior probability) determined from physiological parameter values of patient 4, which may enable patient 4 proactively to seek medical attention prior to receiving instructions for a medical intervention. In this manner, patient 112 may be empowered to take action, as needed, to address his or her peripheral edema status, which may help improve clinical outcomes for patient 112.

In some examples, IMD 104 may be implanted outside of a thoracic cavity of patient 112 (e.g., subcutaneously in the pectoral location illustrated in FIG. 1). In some examples, IMD 104 may be positioned near the sternum near or just below the level of the heart of patient 112, e.g., at least partially within the cardiac silhouette. In some examples, IMD 104 takes the form of the LINQ II^{™} ICM.

In some examples, processing circuitry 118 may use absolute impedance values and statistical representations of impedance values when determining impedance values of an impedance signal. In some examples, absolute impedance values may generally refer to average impedance values. In some examples, processing circuitry 118 may determine an impedance score that indicates one or more physiological parameters of a lymphatic element and determine a peripheral edema status of patient 112 based on the one or more physiological parameters. In some examples, processing circuitry 118 may determine a peripheral edema status of patient 112 based on the one or more physiological parameters based on the impedance score.

In some examples, IMDs 102 may be configured to measure impedance values within the interstitial fluid of patient 112. For example, IMDs 102 may be configured to receive one or more signals indicative of subcutaneous tissue impedance. In some examples, IMDs 102 may be a purely diagnostic device. For example, IMDs 102 may be a device that is to measure subcutaneous impedance values of patient 112.

Subcutaneous impedance may be measured by delivering a signal through an electrical path between electrodes (not shown in FIG. 2). In some examples, the housing of IMD 102 may be used as an electrode in combination with electrodes located on leads. For example, IMD 102 may measure subcutaneous impedance by creating an electrical path between a lead and one of the electrodes. In additional examples, IMD 102 may include an additional lead or lead segment having one or more electrodes positioned subcutaneously or within the subcutaneous layer for measuring subcutaneous impedance. In some examples, two or more electrodes useable for measuring subcutaneous impedance may be formed on or integral with the housing of IMD 102a. In some examples, one or more of the electrodes of IMD 102 contact interstitial fluid.

In some examples, IMD 102 may measure subcutaneous impedance of patient 112 and may process or send impedance data to aggregate evidence of changing impedance that is indicative of one or more physiological parameters of a lymphatic element. The aggregated evidence is referred to as a lymphatic fluid index and may be determined as function of the difference between measured impedance values and reference impedance values. The lymphatic fluid index may then be used to determine a lymphatic fluid level of patient 112 that may help indicate a peripheral edema status of patient 4.

In some examples, IMDs 102 may obtain sensor data every few minutes, hourly, or even daily. Although the techniques for determining a peripheral edema status of a patient based on one or more impedance signals indicative of one or more physiological parameters of a respective lymphatic element, are described herein primarily (e.g., with respect to FIGS. 1-2) as being performed by processing circuitry 118 of computing device 116, such techniques may be performed, in whole or part, by processing circuitry of any one or more devices of system 100, such as processing circuitry 50A of IMD 104, processing circuitry 50 of IMD 102, processing circuitry 118 of computing device 116, and/or processing circuitry of computing system 20.

In some examples, the sensor data is obtained hourly and the processing circuitry 118 may determine the one or more physiological parameters of the lymphatic element based on sensor data, such as impedance signals, aggregated over a period of time, such as a day or during the night while a patient is sleeping to minimize noise from motion artifacts. Processing circuitry 118 may aggregate the lymphatic fluid index values based on the sensor data from IMDs 102 over a period of time, such as an hour, and determine an hourly average or a median of values over a period of time, such as a night. Processing circuitry 118 may aggregate the lymphatic fluid index values over a period of time and determine a lymphatic fluid level or status based at least in part on the aggregated lymphatic fluid index values.

The processing circuitry 118 may aggregate the hourly measurements, while removing noisy measurements, to determine daily measurements that indicate lymphatic fluid indexes which correspond to lymphatic fluid levels. Processing circuitry 118 may aggregate a plurality of lymphatic fluid levels for an average lymphatic fluid value for a period of time, such as an hour, day, or week.

Environment 28 may include computing facilities, e.g., a local network 32, by which computing device 116 and other devices within environment 28 may communicate via network 16, e.g., with HMS 22. For example, environment 28 may be configured with wireless technology, such as IEEE 802.11 wireless networks, IEEE 802.15 ZigBee networks, an ultra-wideband protocol, near-field communication, or the like. Environment 28 may include one or more wireless access points, e.g., wireless access points 34A and 34B (collectively, "wireless access points 34") that provide support for wireless communications throughout environment 28. Additionally or alternatively, e.g., when local network is unavailable, computing device 116 and other devices within environment 28 may be configured to communicate with network 16, e.g., with HMS 22, via a cellular base station 36 and a cellular network.

In some examples, computing device 116 and/or computing system 20 may implement one or more algorithms to evaluate the sensed physiological data received from IMDs 102 and/or IMD 104. In some examples, computing device 116 and/or computing system 20 may have greater processing capacity than IMDs 102 and IMD 104, enabling more complex analysis of the data. In some examples, IMD 104 may have greater processing capacity than IMDs 102. In some examples, computing device 116 and/or computing system 20 may apply the data to a machine learning model or other artificial intelligence developed algorithm, e.g., to determine whether the data is sufficiently indicative of the peripheral edema status, such as whether the patient 112 is suffering from a peripheral edema.

In some examples, HMS 22 may be configured to transmit messages to one or computing devices 38 associated with one or more clinicians 40 via network 16. Care providers may include emergency medical systems (EMS) and hospitals, and may include particular departments within a hospital, such as an emergency department, catheterization lab, or a health response department. Clinician computing devices 38 may include smartphones, desktop, laptop, or tablet computers, or workstations associated with such systems or entities, or employees of such systems or entities. The messages may include any of the data collected by IMDs 102, IMD 104, computing device 116, including sensed physiological data, time of the health condition status, and results of the analysis by IMDs 102, IMD 104, computing device 116, and/or HMS 22. The information transmitted from HMS 22 to clinicians 40 may improve the timeliness and effectiveness of treatment for a peripheral edema of patient 112 by clinicians 40.

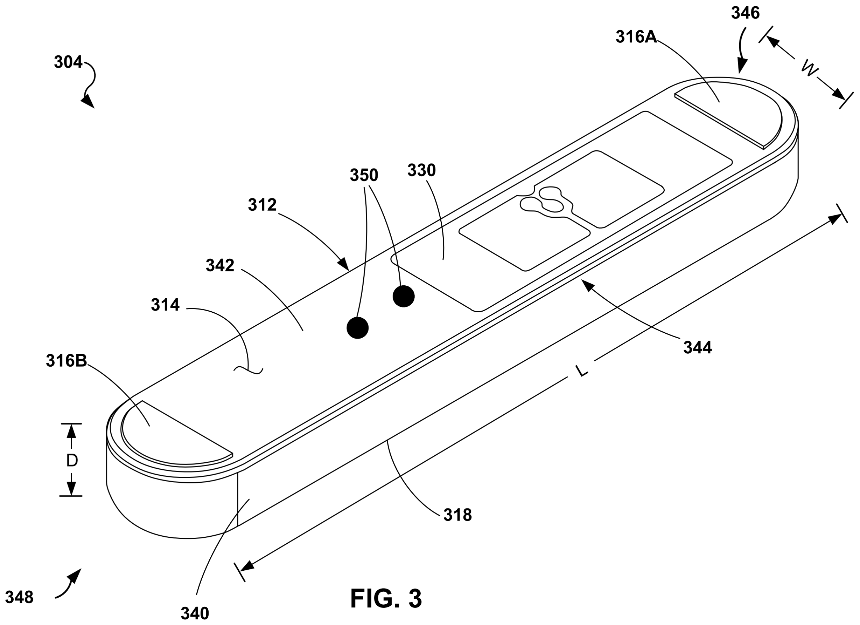

FIG. 3 is a conceptual drawing illustrating an example leadless implantable medical device (IMD) 304, which may be an example configuration of IMD 104 from FIGS. 1 or 2. In some examples, one or more of IMDs 102 may be similarly configured as IMD 304, such as the particular configured of the electrodes of IMD 304, but IMDs 102 may be smaller than IMD 104. In some examples, to be smaller than IMD 104, IMDs 102 may include only those features of IMD 304 necessary or useful for IMDs 102 to be lymphatic focused IMDs which may reduce a size of IMDs 102 which may make IMDs 102 more easily implantable near particular lymphatic elements of interest. In the example shown in FIG. 3, IMD 304 may be embodied as a monitoring device having housing 312, proximal electrode 316A and distal electrode 316B. Housing 312 having a base 340 and an insulative cover 342 may be defined by first major surface 314, second major surface 318, proximal end 346, and distal end 348. Housing 312 encloses electronic circuitry located inside the IMD 304 and protects the circuitry contained therein from body fluids. Housing 312 may be hermetically sealed and configured for subcutaneous implantation. Electrical feedthroughs provide electrical connection of electrodes 316A and 316B.

In the example shown in FIG. 3, IMD 304 is defined by a length *L,* a width *W* and thickness or depth D and is in the form of an elongated rectangular prism wherein the length *L* is much larger than the width *W,* which in turn is larger than the depth *D.* In one example, the geometry of the IMD 304 - in particular a width *W* greater than the depth *D* - is selected to allow IMD 304 to be inserted under the skin of the patient using a minimally invasive procedure and to remain in the desired orientation during insertion. For example, the device shown in FIG. 3 includes radial asymmetries (notably, the rectangular shape) along the longitudinal axis that maintains the device in the proper orientation following insertion. For example, the spacing between proximal electrode 316A and distal electrode 316B may range from 5 millimeters (mm) to 55 mm, 30 mm to 55 mm, 35 mm to 55 mm, and from 40 mm to 55 mm and may be any range or individual spacing from 5 mm to 60 mm. In addition, IMD 304 may have a length L that ranges from 30 mm to about 70 mm. In other examples, the length *L* may range from 5 mm to 60 mm, 40 mm to 60 mm, 45 mm to 60 mm and may be any length or range of lengths between about 30 mm and about 70 mm. In addition, the width *W* of major surface 314 may range from 3 mm to 15, mm, from 3 mm to 10 mm, or from 5 mm to 15 mm, and may be any single or range of widths between 3 mm and 15 mm. The thickness of depth *D* of IMD 304 may range from 2 mm to 15 mm, from 2 mm to 9 mm, from 2 mm to 5 mm, from 5 mm to 15 mm, and may be any single or range of depths between 2 mm and 15 mm. In addition, IMD 304 according to an example of the present disclosure is has a geometry and size designed for ease of implant and patient comfort. Examples of IMD 304 described in this disclosure may have a volume of three cubic centimeters (cm) or less, 1.5 cubic cm or less or any volume between three and 1.5 cubic centimeters.

In some examples, in examples in which IMDs 102 are configured similarly to IMD 304, IMDs may have a volume of one cubic cm or less. In some examples, IMDs 102 may have a length L that ranges from 5 mm to 20 mm. In addition, the width *W* of major surface 314 of IMD 102 may range from 2 mm to 10 mm, and the depth *D* of IMD 304 may range from 2 mm to 5 mm.

In the example shown in FIG. 3, once inserted within the patient, the first major surface 314 faces outward, toward the skin of the patient while the second major surface 318 is located opposite the first major surface 314. In addition, in the example shown in FIG. 3, proximal end 346 and distal end 348 are rounded to reduce discomfort and irritation to surrounding tissue once inserted under the skin of the patient.

Proximal electrode 316A is at or proximate to proximal end 346, and distal electrode 316B is at or proximate to distal end 348. Proximal electrode 316A and distal electrode 316B may be used to sense cardiac ECG signals. Cardiac signals may be stored in a memory of IMD 304, and data may be transmitted via integrated antenna 330 to another device, which may be another implantable device or an external device, such as computing device 116. In some example, electrodes 316A and 316B may additionally or alternatively be used for sensing any bio-potential signal of interest, which may be, for example, an ECG, EEG, or a nerve signal, or for measuring impedance, from any implanted location.

In the example shown in FIG. 3, proximal electrode 316A is at or in close proximity to the proximal end 346 and distal electrode 316B is at or in close proximity to distal end 348. In this example, distal electrode 316B is not limited to a flattened, outward facing surface, but may extend from first major surface 314 around rounded edges and/or end surface and onto the second major surface 318 so that the electrode 316B has a three-dimensional curved configuration. In some examples, electrode 316B is an uninsulated portion of a metallic, e.g., titanium, part of housing 312.

In the example shown in FIG. 3, proximal electrode 316A is located on first major surface 314 and is substantially flat, and outward facing. However, in other examples proximal electrode 316A may utilize the three-dimensional curved configuration of distal electrode 316B, providing a three-dimensional proximal electrode (not shown in this example). Similarly, in other examples distal electrode 316B may utilize a substantially flat, outward facing electrode located on first major surface 314 similar to that shown with respect to proximal electrode 316A.

The various electrode configurations allow for configurations in which proximal electrode 316A and distal electrode 316B are located on both first major surface 314 and second major surface 318. In other configurations, such as that shown in FIG. 3, only one of proximal electrode 316A and distal electrode 316B is located on both major surfaces 314 and 318, and in still other configurations both proximal electrode 316A and distal electrode 316B are located on one of the first major surface 314 or the second major surface 318 (e.g., proximal electrode 316A located on first major surface 314 while distal electrode 316B is located on second major surface 318). In another example, IMD 304 may include electrodes on both major surface 314 and 318 at or near the proximal end and distal end 348 of the device, such that a total of four electrodes are included on IMD 304. Electrodes 316A and 316B may be formed of a plurality of different types of biocompatible conductive material, e.g. stainless steel, titanium, platinum, iridium, or alloys thereof, and may utilize one or more coatings such as titanium nitride or fractal titanium nitride.

Integrated antenna 330 allows IMD 304 to transmit and/or receive data. In other examples, integrated antenna 330 may be formed on the opposite major surface as proximal electrode 316A, or may be incorporated within the housing 312 of IMD 304. Anti-migration projections may be located adjacent to integrated antenna 330 and may protrude away from first major surface 314 to prevent longitudinal movement of the device. Anti-migration projections may include a plurality (e.g., nine) small bumps or protrusions extending away from first major surface 314. IMD 304 may include suture holes, which provides another means of securing IMD 304 to the patient to prevent movement following insertion. Suture holes may be located adjacent to proximal electrode 316A.

Various circuitries and components of IMD 304, e.g., processing circuitry or other circuitries, may be formed or placed on an inner surface of cover 342, or within base 340. In some examples, a battery or other power source of IMD 304 may be included within base 340. In the illustrated example, antenna 330 is formed or placed on the outer surface of cover 342, but may be formed or placed on the inner surface in some examples. In some examples, insulative cover 342 may be positioned over an open base 340 such that base 340 and cover 342 enclose the circuitries and other components and protect them from fluids such as body fluids. Housing 312 including base 340 and insulative cover 342 may be hermetically sealed and configured for subcutaneous implantation.

Circuitries and components may be formed on the inner side of insulative cover 342, such as by using flip-chip technology. Insulative cover 342 may be flipped onto a base 340. When flipped and placed onto base 340, the components of IMD 304 formed on the inner side of insulative cover 342 may be positioned in a gap 344 defined by base 340. Electrodes 316A and 316B and antenna 330 may be electrically connected to circuitry formed on the inner side of insulative cover 342 through one or more vias (not shown) formed through insulative cover 342. Insulative cover 342 may be formed of sapphire (i.e., corundum), glass, ceramic, parylene, and/or any other suitable insulating material. Base 340 may be formed from titanium or any other suitable material (e.g., a biocompatible material). Electrodes 316A and 316B may be formed from any of stainless steel, titanium, tantalum, niobium, platinum, iridium, or alloys thereof. In addition, electrodes 316A and 316B may be coated with a material such as titanium nitride or fractal titanium nitride, although other suitable materials and coatings for such electrodes may be used.

IMD 304 may further include one or more sensors 350. Sensors 350 may include optical, impedance, temperature, or any other type of sensor which may be configured to sense physiological signals. Sensors 350 may be impedance sensors, temperature sensors, and/or optical sensors, and processing circuitry of IMD 304 or other devices that communicate with IMD 304 may determine a peripheral edema status based on signals from sensors 350.

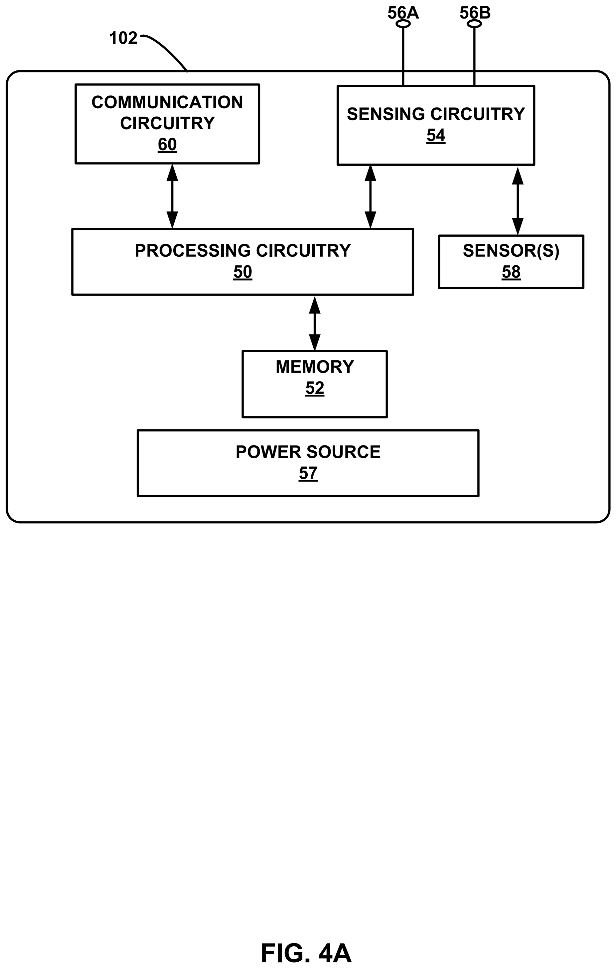

FIG. 4A is a block diagram illustrating an example configuration of a respective IMD 102 of FIGS. 1-2. As shown in FIG. 4A, IMD 102 includes processing circuitry 50, memory 52, sensing circuitry 54 coupled to electrodes 56A and 56B (hereinafter, "electrodes 56") and one or more sensor(s) 58, power source 57, and communication circuitry 60.

Processing circuitry 50 may include fixed function circuitry and/or programmable processing circuitry. Processing circuitry 50 may include any one or more of a microprocessor, a controller, a graphics processing unit (GPU), a tensor processing unit (TPU), a digital signal processor (DSP), an application specific integrated circuit (ASIC), a field-programmable gate array (FPGA), or equivalent discrete or analog logic circuitry. In some examples, processing circuitry 50 may include multiple components, such as any combination of one or more microprocessors, one or more controllers, one or more GPUs, one or more TPUs, one or more DSPs, one or more ASICs, or one or more FPGAs, as well as other discrete or integrated logic circuitry. The functions attributed to processing circuitry 50 herein may be embodied as software, firmware, hardware, or any combination thereof. In some examples, memory 52 includes computer-readable instructions that, when executed by processing circuitry 50, cause IMD 102 and processing circuitry 50 to perform various functions attributed herein to IMD 102 and processing circuitry 50. Memory 52 may include any volatile, non-volatile, magnetic, optical, or electrical media, such as a random-access memory (RAM), read-only memory (ROM), non-volatile RAM (NVRAM), electrically-erasable programmable ROM (EEPROM), flash memory, or any other digital media.

Sensing circuitry 54 may monitor signals from electrodes 56 in order to, for example, monitor impedance signals indicative of one or more physiological parameters of a respective lymphatic element of patient 112 and produce corresponding impedance data for patient 112. In some examples, processing circuitry 50 may identify features of the sensed impedance signals to determine a peripheral edema status of the patient 112. Processing circuitry 50 may store the digitized impedance signals and features of the impedance signals used to determine the one or more physiological parameters of the lymphatic element in memory 52 as episode data for the detected peripheral edema.

In some examples, sensing circuitry 54 measures impedance, e.g., of a respective lymphatic element 113 proximate to a respective IMD 102, via electrodes 56. In some examples, lymphatic element 113 may include a lymph node and/or lymph vessel of patient 112. The measured impedance may vary based on a degree of peripheral edema. Processing circuitry 50 may determine physiological data relating to peripheral edema based on the measured impedance, such as at least one of afferent lymph flow, efferent lymph flow, lymph node volume, lymph node geometric size, lymph node arterial waveform, lymph node arterial pressure, lymph node activity, inflammatory response, and/or cytokine release. In some examples, IMDs 102 may provide electrostimulation via one or more electrodes 56. In some examples, IMDs 102 may sense physiological signals, such as impedance signals indicative of one or more physiological parameters of a respective lymphatic element and provide electrostimulation via one or more electrodes 56. In some examples, IMDs 102 may sense physiological signals via one or more electrodes 56 and provide electro stimulation via one or more different electrodes of electrodes 56 than the electrodes that sense physiological signals. In some examples, IMDs 102 may sense physiological signals and provide electrostimulation via the same one or more electrodes 56.

In some examples, IMD 102 includes additional sensors 58, such as an optical sensor. In some examples, sensing circuitry 54 may include one or more filters and amplifiers for filtering and amplifying signals received from one or more of electrodes 56 and/or sensors 58. In some examples, sensing circuitry 54 and/or processing circuitry 50 may include a rectifier, filter and/or amplifier, a sense amplifier, comparator, and/or analog-to-digital converter. Processing circuitry 50 may determine physiological data, e.g., values of physiological parameters of patient 112, based on signals from sensors 58, which may be stored in memory 52. In some examples, power source 57 may include a battery. In some examples, power source 57 may include a rechargeable battery. In some examples, power source 57 may include at least one capacitor. In some examples, power source may include one or more of capacitors and/or batteries. In some examples, IMDs 102 being of a reduced size in comparison to IMD 104 to be configured to lymphatic focused IMDs a size of power source 57 may be smaller in comparison to IMD 104.

Communication circuitry 60 may include any suitable hardware, firmware, software, or any combination thereof for wirelessly communicating with another device, such as other IMDs 102, IMD 104, and/or computing device 116.

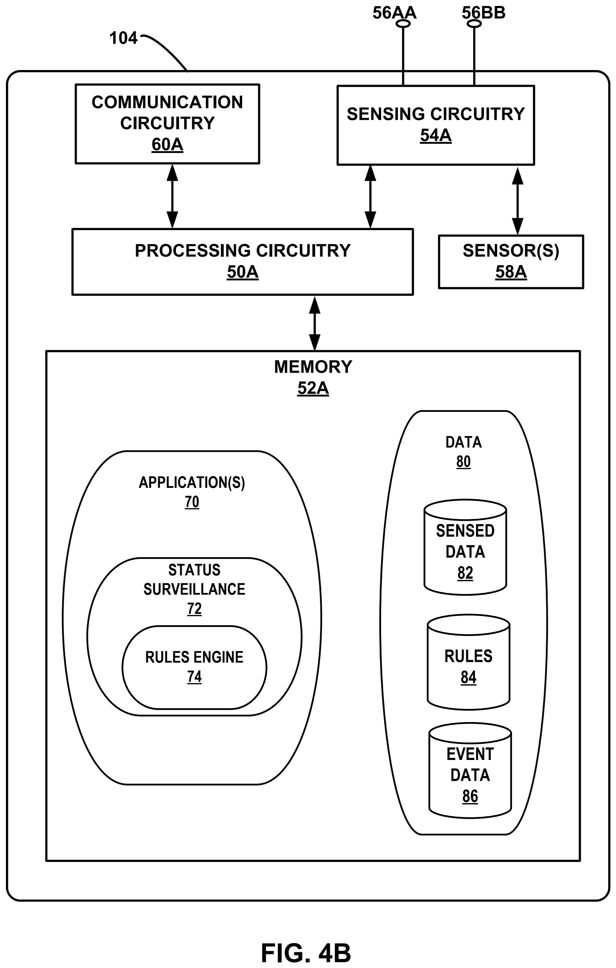

FIG. 4B is a block diagram illustrating an example configuration of IMD 102 4 of FIGS. 1-3. As shown in FIG. 4B, IMD 104 includes processing circuitry 50A, memory 52A, sensing circuitry 54A coupled to electrodes 56AA and 56BB (hereinafter, "electrodes 56T") and one or more sensor(s) 58A, and communication circuitry 60A. While not shown in FIG. 4B, IMD 104 may include a power source (e.g., capacitors and batteries).

Processing circuitry 50A may include fixed function circuitry and/or programmable processing circuitry. Processing circuitry 50A may include any one or more of a microprocessor, a controller, a graphics processing unit (GPU), a tensor processing unit (TPU), a digital signal processor (DSP), an application specific integrated circuit (ASIC), a field-programmable gate array (FPGA), or equivalent discrete or analog logic circuitry. In some examples, processing circuitry 50A may include multiple components, such as any combination of one or more microprocessors, one or more controllers, one or more GPUs, one or more TPUs, one or more DSPs, one or more ASICs, or one or more FPGAs, as well as other discrete or integrated logic circuitry. The functions attributed to processing circuitry 50A herein may be embodied as software, firmware, hardware, or any combination thereof. In some examples, memory 52A includes computer-readable instructions that, when executed by processing circuitry 50A, cause IMD 104 and processing circuitry 50A to perform various functions attributed herein to IMD 104 and processing circuitry 50A. Memory 52A may include any volatile, non-volatile, magnetic, optical, or electrical media, such as a random-access memory (RAM), read-only memory (ROM), non-volatile RAM (NVRAM), electrically-erasable programmable ROM (EEPROM), flash memory, or any other digital media.

Sensing circuitry 54A may monitor signals from electrodes 56T in order to, for example, monitor electrical activity of a heart of patient 4 and produce ECG data for patient 112. In some examples, communication circuitry 60A may receive one or more impedance signals indicative of one or more physiological parameters of a respective lymphatic element from one or more IMDs 102. In some examples, communication circuitry 60A may receive one or more physiological parameters of a respective lymphatic element from one or more IMDs 102. In some examples, processing circuitry 50A may determine the one or more physiological parameters of respective lymphatic elements based on the received impedance signals. In some examples, processing circuitry 50A may determine a peripheral edema status of the patient based on the one or more physiological parameters and may output an indication of the peripheral edema status. Processing circuitry 50A may store the digitized impedance signals and features of the impedance signals used to determine the one or more physiological parameters of the lymphatic element in memory 52 as episode data for the detected peripheral edema. In some examples, processing circuitry 50A may store the one or more physiological parameters of the respective lymphatic elements used to determine a peripheral edema status of the patient 112.

In some examples, sensing circuitry 54A measures impedance, e.g., of tissue proximate to IMD 10, via electrodes 56T. The measured impedance may vary based on respiration and a degree of perfusion or edema. Processing circuitry 50A may determine physiological data relating to respiration, perfusion, and/or edema based on the measured impedance. In some examples, IMD 104 may provide electrostimulation via one or more electrodes 56T. In some examples, IMD 104 may sense physiological signals and provide electrostimulation via one or more electrodes 56T. In some examples, IMD 104 may sense physiological signals via one or more electrodes 56T and provide electrostimulation via one or more different electrodes of electrodes 56T. In some examples, IMD 104 may sense physiological signals and provide electrostimulation via the same one or more electrodes 56T.

In some examples, IMD 104 includes sensors 58A, such as one or more accelerometers, microphones, optical sensors, temperature sensors, and/or pressure sensors. In some examples, sensing circuitry 54A may include one or more filters and amplifiers for filtering and amplifying signals received from one or more of electrodes 56T and/or sensors 58A. In some examples, sensing circuitry 54 and/or processing circuitry 50 may include a rectifier, filter and/or amplifier, a sense amplifier, comparator, and/or analog-to-digital converter. Processing circuitry 50A may determine physiological data, e.g., values of physiological parameters of patient 112, based on signals from sensors 58A, which may be stored in memory 52A.

Memory 52A may store applications 70 executable by processing circuitry 50A, and data 80. Applications 70 may include an acute health event, such as peripheral edema, surveillance application 72. Processing circuitry 50A may execute event surveillance application 72 to detect a peripheral edema event of patient 112 based on combination of one or more of the types of physiological data described herein, which may be stored as sensed data 82. In some examples, sensed data 82 may additionally include data sensed by other devices, e.g., IMDs 102, and received via communication circuitry 60A. Event surveillance application 72 may be configured with a rules engine 74. Rules engine 74 may apply rules 84 to sensed data 82. Rules 84 may include one or more models, algorithms, decision trees, and/or thresholds. In some cases, rules 84 may be developed based on machine learning.

As examples, event surveillance application 72 may detect a peripheral edema status of patient 112 (FIGS. 1-2). When event surveillance application 72 detects a peripheral edema, event surveillance application 72 may store the sensed data 82 that lead to the detection (and in some cases a window of data preceding and/or following the detection) as event data 86.

In some examples, in response to detection of a peripheral edema, processing circuitry 50A may attempt to transmit, via communication circuitry 60A, event data 86 for the event to computing device 116. This transmission may be included in a message indicating the peripheral edema and may include a request for an indication from computing device 116 that the message including the peripheral edema event has been received and that the computing device 116 has network 16 access.

Communication circuitry 60A may include any suitable hardware, firmware, software, or any combination thereof for wirelessly communicating with another device, such as IMDs 102 and/or computing device 116.

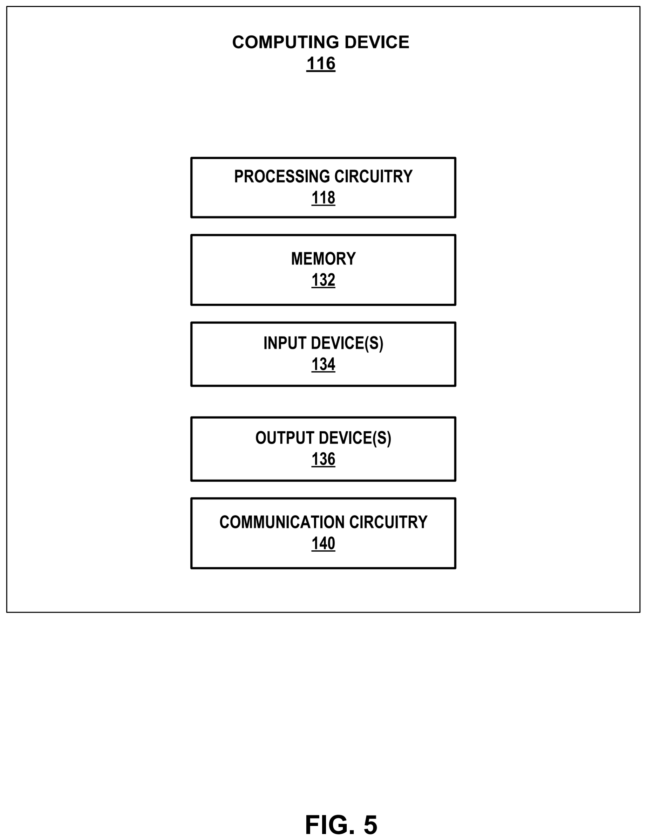

FIG. 5 is a block diagram illustrating an example configuration of a computing device 116. In some examples, computing device 116 takes the form of a smartphone, a laptop, a tablet computer, a personal digital assistant (PDA), a smartwatch or other wearable computing device.

As shown in FIG. 5, computing device 116 includes processing circuitry 118, memory 132, one or more input devices 134, one or more output devices 136, and communication circuitry 140. Although shown in FIG. 5 as a stand-alone device for purposes of example, computing device 116 may be any component or system that includes processing circuitry or other suitable computing environment for executing software instructions and, for example, need not necessarily include one or more elements shown in FIG. 5.

Processing circuitry 118 is configured to implement functionality and/or process instructions for execution within computing device 116. Examples of processing circuitry 118 may include, any one or more microprocessors, controllers, GPUs, TPUs, DSPs, ASICs, FPGAs, or equivalent discrete or integrated logic circuitry.

Memory 132 may be configured to store information within computing device 116, for processing during operation of computing device 14. Memory 132, in some examples, is described as a computer-readable storage medium. In some examples, memory 132 includes a temporary memory or a volatile memory. Examples of volatile memories include random access memories (RAM), dynamic random access memories (DRAM), static random access memories (SRAM), and other forms of volatile memories known in the art. Memory 132, in some examples, also includes one or more memories configured for long-term storage of information, e.g. including non-volatile storage elements. Examples of such non-volatile storage elements include magnetic hard discs, optical discs, floppy discs, flash memories, or forms of electrically programmable memories (EPROM) or electrically erasable and programmable (EEPROM) memories.

One or more input devices 134 of computing device 116 may receive input, e.g., from a user. Examples of input are tactile, audio, kinetic, and optical input. Input devices 134 may include, as examples, a mouse, keyboard, voice responsive system, camera, buttons, control pad, microphone, presence-sensitive or touch-sensitive component (e.g., screen), or any other device for detecting input from a user or a machine. In some examples, input devices 134 may additionally or alternatively include physiologic sensors, such as photoplethysmogram (PPG) sensors, ECG sensors, temperature sensors, impedance sensors, etc., that may augment and/or aid in the diagnosis of a peripheral edema status of patient 112.

One or more output devices 136 of computing device 116 may generate output, e.g., to patient 112 or another user. Examples of output are tactile, audio, and visual output. Output devices 136 of computing device 116 may include a presence-sensitive screen, sound card, video graphics adapter card, speaker, cathode ray tube (CRT) monitor, liquid crystal display (LCD), light emitting diodes (LEDs), or any type of device for generating tactile, audio, and/or visual output.

Communication circuitry 140 of computing device 116 may communicate with other devices by transmitting and receiving data. For example, communication circuitry 140 may be configured to communicate with IMDs 102, IMD 104, and/or computing system 20. Communication circuitry 140 may include a network interface card, such as an Ethernet card, an optical transceiver, a radio frequency transceiver, or any other type of device that can send and receive information. For example, communication circuitry 140 may include a radio transceiver configured for communication according to standards or protocols, such as 3G, 4G, 5G, WiFi (e.g., 802.11 or 802.15 ZigBee), Bluetooth^{®}, Bluetooth^{®} Low Energy (BLE), or forms of satellite communication.

In some examples, communication circuitry 140 may receive one or more impedance signals indicative of one or more physiological parameters of a respective lymphatic element from one or more IMDs 102 and/or IMD 104. In some examples, communication circuitry 140 may receive one or more physiological parameters of a respective lymphatic element from one or more IMDs 102 and/or IMD 104. In some examples, processing circuitry 118 may determine the one or more physiological parameters of respective lymphatic elements based on the received impedance signals. In some examples, processing circuitry 118 may determine a peripheral edema status of the patient based on the one or more physiological parameters and may output an indication of the peripheral edema status. Processing circuitry 118 may store the digitized impedance signals and features of the impedance signals used to determine the one or more physiological parameters of the lymphatic element in memory 132 as episode data for the detected peripheral edema. In some examples, processing circuitry 118 may store the one or more physiological parameters of the respective lymphatic elements used to determine a peripheral edema status of the patient 112.

Although the techniques for determining a peripheral edema status of a patient based on one or more impedance signals indicative of one or more physiological parameters of a respective lymphatic element, are described herein primarily (e.g., with respect to FIGS. 1-2) as being performed by processing circuitry 118 of computing device 116, such techniques may be performed, in whole or part, by processing circuitry of any one or more devices of system 100, such as processing circuitry 50A of IMD 104, processing circuitry 50 of IMD 102, processing circuitry 118 of computing device 116, and/or processing circuitry of computing system 20.

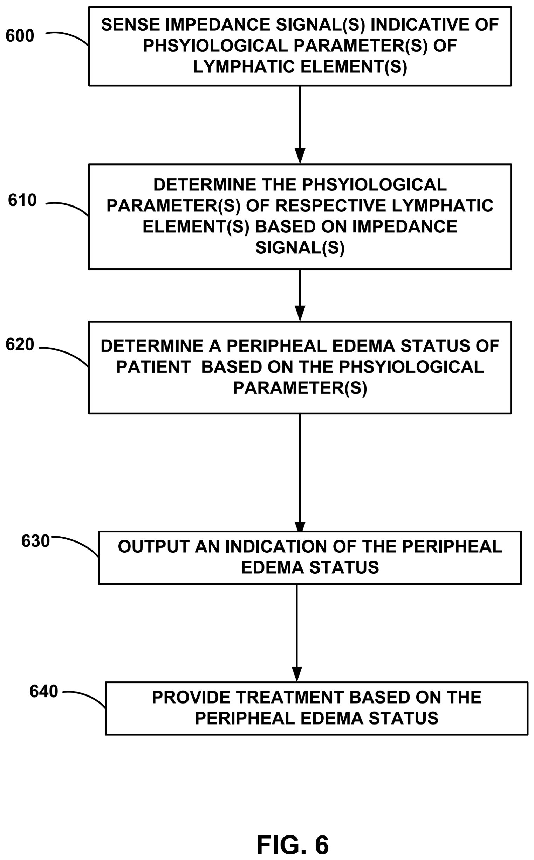

FIG. 6 is a flow diagram illustrating an example technique for operation of a medical system 100 to determine a peripheral edema status of patient 112. As indicated by FIG. 6, one or more lymphatic focused IMDs 102 may sense a respective impedance signal indicative of one or more physiological parameters of a respective lymphatic element 113 of patient 112 (600). In some examples, one or more of lymphatic focused IMDs 102 may be configured for subcutaneous implantation proximate a respective lymphatic element of patient 112. In some examples, one or more of lymphatic focused IMDs 102 may be configured to sense impedance signal(s) via a plurality of electrodes. In some examples, the impedance signal may include at least one of real impedance and/or reactive impedance.

Processing circuitry 118 may determine the one or more physiological parameters of the lymphatic element(s) based on the respective impedance signal(s) respectively sensed via the plurality of electrodes of the one or more lymphatic focused IMDs 102 (610). In some examples, the physiological parameters may include at least one of afferent lymph flow, efferent lymph flow, lymph node volume, lymph node geometric size, lymph node arterial waveform, lymph node arterial pressure, lymph node activity of the respective lymphatic element. In some examples, the physiological parameters may additionally or alternatively include inflammatory response and/or cytokine release.

Processing circuitry 118 may determine a peripheral edema status of patient 112 based on the one or more physiological parameters (620). In some examples, a peripheral edema status may include one or more of an indication of whether a patient is suffering from a peripheral edema, an indication of whether a peripheral edema is changing, such as worsening or improving, over a period of time, whether a peripheral edema indicates treatment and/or a change in treatment be administered. In some examples, processing circuitry 118 may determine a location of a peripheral edema of patient 112 based on the one or more physiological parameters that are respectively based on one or more sensed signals, such as impedance, optical signal, and/or temperature, sensed via one or more IMDs 102.

In some examples, processing circuitry 118 may output an indication of the peripheral edema status (630). In some examples, processing circuitry 118 may be configured to output an indication of the peripheral edema status that may facilitate determinations of patient wellness, determination of severity of peripheral edema, and/or determination of risk of suffering severe peripheral edema (e.g., such as 10% or more weight gain) in the near future, such as within the next 7 days, within the next 14 days, within the next month, etc., and may lead to clinical interventions to suppress peripheral edema such as medications and/or compression garments.

In some examples, processing circuitry 118 may be configured to provide treatment, such as electro stimulation, based on a determination of a peripheral edema status of patient 112 (640). In some examples, processing circuitry 118 may be configured to provide and/or adjust treatment, such as electrostimulation, based on a determination of whether treatment being administered to patient 112 is effective and/or ineffective. In some examples, processing circuitry 118 may determine a particular treatment to administer to patient, such as type and/or amount of treatment, based on the determined location of the peripheral edema. In some examples, processing circuitry 118 may determine one or more particular IMDs 102 to provide stimulation treatment in response to the determined location of the peripheral edema. For example, processing circuitry 118 may determine to have one or more IMDs 102 that are within a particular distance threshold of the determined location of the peripheral edema to provide stimulation treatment to treat the peripheral edema.

In some examples, processing circuitry 118 may utilize machine learning, such as a deep learning algorithm or model (e.g., a neural network or deep belief network), to determine a peripheral edema status based on impedance signal(s), physiological parameters of the lymphatic element, and/or, in some examples, one or more additional parameters of patient 112. Processing circuitry 118 may be configured to execute an artificial intelligence (AI) engine that operates according to one or more models, such as machine learning models. Machine learning models may include any number of different types of machine learning models, such as neural networks, deep neural networks, convolution neural networks, recurrent neural networks, such as long short term memory networks, dense neural networks, and the like. In some examples, various feature inputs to the AI engine may be fed as direct inputs to different layers in a network and not necessarily prior to the convolution layers. Although described with respect to machine learning models, the techniques described in this disclosure are also applicable to other types of AI models, including rule-based models, finite state machines, and the like. For example, the techniques described in this disclosure are also applicable to Bayesian Belief Networks (BBN) or Bayesian machine learning models (these sometimes referred to as Bayesian Networks or Bayesian frameworks herein), Markov random fields, graphical models, AI models (e.g., Naive Bayes classifiers or deep learning models), and/or other belief networks, such as sigmoid belief networks, deep belief networks (DBNs), etc. In other examples, the disclosed technology may leverage non-Bayesian prediction or probability modeling, such as frequentist inference modeling or other statistical models.

Machine learning may generally enable a computing device to analyze input data and identify an action to be performed responsive to the input data. Each machine learning model may be trained using training data that reflects likely input data. The training data may be labeled or unlabeled (meaning that the correct action to be taken based on a sample of training data is explicitly stated or not explicitly stated, respectively).

The training of the machine learning model may be guided (in that a designer, such as a computer programmer, may direct the training to guide the machine learning model to identify the correct action in view of the input data) or unguided (in that the machine learning model is not guided by a designer to identify the correct action in view of the input data). In some instances, the machine learning model is trained through a combination of labeled and unlabeled training data, a combination of guided and unguided training, or possibly combinations thereof. Examples of machine learning include nearest neighbor, naive Bayes, decision trees, linear regression, support vector machines, neural networks, k-Means clustering, Q-learning, temporal difference, deep adversarial networks, evolutionary algorithms or other supervised, unsupervised, semi-supervised, or reinforcement learning algorithms to train one or more models.

Processing circuitry 118 may utilize machine learning, such as a deep learning algorithm or model (e.g., a neural network or deep belief network), to determine a peripheral edema status based, at least in part, on sensed impedance signal(s) indicative of one or more physiological parameters of a respective lymphatic element. Processing circuitry 118 may train a deep learning model to represent a relationship of the impedance signal(s) to a peripheral edema status. For example, processing circuitry 118 may train the deep learning model using impedance signals from other patients. In some examples, processing circuitry 118 may train the deep leaning model by adjusting the weights of a hidden layer of a neural network model to balance the contribution of each input (e.g., characteristics of the respective impedance signals) according to determining a peripheral edema status. Once the deep learning model is trained, processing circuitry 118 may obtain and apply data, such as the sensed impedance signal(s) indicative of one or more physiological parameters of a respective lymphatic element and/or the one or more physiological parameters of respective lymphatic elements, to the trained deep learning model to determine a peripheral edema status of patient 112.

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as clinician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, DRAM, SRAM, magnetic discs, optical discs, flash memories, or forms of EPROM or EEPROM. The instructions may be executed to support one or more aspects of the functionality described in this disclosure.

In addition, in some respects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components or integrated within common or separate hardware or software components. Also, the techniques could be fully implemented in one or more circuits or logic elements. The techniques of this disclosure may be implemented in a wide variety of devices or apparatuses, including an IMD, an external programmer, a combination of an IMD and external programmer, an integrated circuit (IC) or a set of ICs, and/or discrete electrical circuitry, residing in an IMD and/or external programmer.

This disclosure describes each of the following examples.

Example 1: A system includes an implantable medical device (IMD) includes a housing configured for subcutaneous implantation proximate a lymphatic element of a patient; and a plurality of electrodes configured to sense an impedance signal indicative of one or more physiological parameters of the lymphatic element; and processing circuitry configured to: determine the one or more physiological parameters of the lymphatic element based on the impedance signal; determine a peripheral edema status of the patient based on the one or more physiological parameters; and output an indication of the peripheral edema status.

Example 2: The system of example 1, wherein the IMD is configured to be implanted in a limb of the patient.

Example 3: The system of any of examples 1-2, wherein the impedance signal includes at least one of real impedance or reactive impedance.

Example 4: The system of any of examples 1-3, wherein the physiological parameters include at least one of afferent lymph flow, efferent lymph flow, lymph node volume, lymph node geometric size, lymph node arterial waveform, lymph node arterial pressure, lymph node activity, inflammatory response, and/or cytokine release.

Example 5: The system of any of examples 1-4, wherein the lymphatic element includes at least one of a lymph node or a lymph vessel of the patient.

Example 6: The system of any of examples 1-5, wherein the IMD is a first IMD, the lymphatic element is a first lymphatic element, the physiological parameters are first physiological parameters of the first lymphatic element, the plurality of electrodes are a first plurality of electrodes, and the impedance signal is a first impedance signal, and wherein the system further comprises a second IMD includes a second housing configured for subcutaneous implantation proximate a second lymphatic element of the patient; and a second plurality of electrodes configured to sense a second impedance signal indicative of one or more second physiological parameters of the second lymphatic element.

Example 7: The system of example 6, wherein the processing circuitry is further configured to: determine one or more second physiological parameters based on the second impedance signal; and determine the peripheral edema status based on the one or more first physiological parameters and the one or more second physiological parameters.

Example 8: The system of any of examples 1-7 further comprising a computing device, wherein the computing device includes the processing circuitry.

Example 9: The system of example 8, wherein the computing device is separate from the first IMD and the second IMD, and the processing circuitry is configured to receive the first impedance signal via communication circuitry of the first IMD and receive the second impedance signal via communication circuitry of the second IMD.

Example 10: The system of any of examples 8-9, wherein the computing device is positioned in a third IMD.

Example 11: The system of any of examples 8-9, wherein the computing device is external with respect to the patient.

Example 12: The system of any of examples 6-11, wherein the first IMD and the second IMD are each respectively configured to be implanted in a limb of the patient.

Example 13: The system of any of examples 6-12, wherein the first impedance signal includes at least one of real impedance or reactive impedance and the second impedance signal includes at least one of real impedance or reactive impedance.

Example 14: The system of any of examples 6-13, wherein the first physiological parameters include at least one of afferent lymph flow, efferent lymph flow, lymph node volume, lymph node geometric size, lymph node arterial waveform, lymph node arterial pressure, and/or lymph node activity of the first lymphatic element.

Example 15: The system of example 14, wherein the second physiological parameters include at least one of afferent lymph flow, efferent lymph flow, lymph node volume, lymph node geometric size, lymph node arterial waveform, lymph node arterial pressure, and/or lymph node activity of the second lymphatic element.

Example 16: The system of any of examples 6-15, wherein the first lymphatic element includes at least one of a first lymph node or a first lymph vessel of the patient, and the second lymphatic element includes at least one of a first lymph node or a second lymph vessel of the patient.

Example 17: The system of any of examples 6-16, wherein the first IMD further comprises a first optical sensor configured to sense a first optical signal, the second IMD further comprises a second optical sensor configured to sense a second optical signal, and the processing circuitry is further configured to: determine at least one of first lymph node arterial waveform or first lymph node arterial pressure based on the first optical signal; determine at least one of second lymph node arterial waveform or second lymph node arterial pressure based on the second optical signal; and determine the peripheral edema status based on the one or more first physiological parameters, the one or more second physiological parameters, the at least one of first lymph node arterial waveform or first lymph node arterial pressure, and the at least one of second lymph node arterial waveform or second lymph node arterial pressure.

Example 18: The system of any of examples 1-17, wherein the IMD is configured to provide electrostimulation based on the peripheral edema status.

Example 19: The system of any of examples 6-17, wherein the second IMD is configured to provide electrostimulation based on the peripheral edema status.

Example 20: The system of any of examples 1-17 further comprising an electrostimulation device configured to provide electrostimulation based on the peripheral edema status.

Example 21: A method of determining a peripheral edema status of a patient includes sensing, via an implantable medical device (IMD) configured for subcutaneous implantation proximate a lymphatic element of a patient, an impedance signal indicative of one or more physiological parameters of the lymphatic element; determining, via processing circuitry, the one or more physiological parameters of the lymphatic element based on the impedance signal; determining, via the processing circuitry, a peripheral edema status of the patient based on the one or more physiological parameters; and outputting, via the processing circuitry, an indication of the peripheral edema status.

Example 22: The method of example 21, wherein the IMD is configured to be implanted in a limb of the patient.

Example 23: The method of any of examples 21-22, wherein the impedance signal includes at least one of real impedance or reactive impedance.

Example 24: The method of any of examples 21-23 wherein the physiological parameters include at least one of afferent lymph flow, efferent lymph flow, lymph node volume, lymph node geometric size, lymph node arterial waveform, lymph node arterial pressure, lymph node activity, inflammatory response, and/or cytokine release.

Example 25: The method of any of examples 21-24, wherein the lymphatic element includes at least one of a lymph node or a lymph vessel of the patient.

Example 26: The method of any of examples 21-25, wherein the IMD is a first IMD, the lymphatic element is a first lymphatic element, the physiological parameters are first physiological parameters of the first lymphatic element, and the impedance signal is a first impedance signal, the method further includes sensing, via a second IMD configured for subcutaneous implantation proximate a second lymphatic element of the patient, a second impedance signal indicative of one or more second physiological parameters of the second lymphatic element.

Example 27: The method of example 26 further includes determining, via the processing circuitry, one or more second physiological parameters based on the second impedance signal; and determining, via the processing circuitry, the peripheral edema status based on the one or more first physiological parameters and the one or more second physiological parameters.

Example 28: The method any of examples 21-27, wherein the processing circuitry is positioned in a computing device.

Example 29: The method of example 28, wherein the computing device is separate from the first IMD and the second IMD, the method further includes receiving, by the computing device, the first impedance signal via communication circuitry of the first IMD; and receiving, by the computing device, the second impedance signal via communication circuitry of the second IMD.

Example 30: The method of any of examples 28-29, wherein the computing device is positioned in a third IMD.

Example 31: The method of any of examples 28-29, wherein the computing device is external with respect to the patient.

Example 32: The method of any of examples 26-31, wherein the first IMD and the second IMD are each respectively configured to be implanted in a limb of the patient.

Example 33: The method of any of examples 26-32, wherein the first impedance signal includes at least one of real impedance or reactive impedance and the second impedance signal includes at least one of real impedance or reactive impedance.

Example 34: The method of any of examples 26-33, wherein the first physiological parameters include at least one of afferent lymph flow, efferent lymph flow, lymph node volume, lymph node geometric size, lymph node arterial waveform, lymph node arterial pressure, and/or lymph node activity of the first lymphatic element.

Example 35: The method of example 34, wherein the second physiological parameters include at least one of afferent lymph flow, efferent lymph flow, lymph node volume, lymph node geometric size, lymph node arterial waveform, lymph node arterial pressure, and/or lymph node activity of the second lymphatic element.

Example 36: The method of any of examples 26-35, wherein the first lymphatic element includes at least one of a first lymph node or a first lymph vessel of the patient, and the second lymphatic element includes at least one of a first lymph node or a second lymph vessel of the patient.

Example 37: The method of any of examples 26-36 further includes sensing, by a first optical sensor of the first IMD, a first optical signal; sensing, by a second optical sensor of the second IMD, a second optical signal; determining, by the processing circuitry, at least one of first lymph node arterial waveform or first lymph node arterial pressure based on the first optical signal; determining, by the processing circuitry, at least one of second lymph node arterial waveform or second lymph node arterial pressure based on the second optical signal; and determining, by the processing circuitry, the peripheral edema status based on the one or more first physiological parameters, the one or more second physiological parameters, the at least one of first lymph node arterial waveform or first lymph node arterial pressure, and the at least one of second lymph node arterial waveform or second lymph node arterial pressure.

Example 38: The method of any of examples 21-37 further comprising providing, via the IMD, electrostimulation to the patient based on the peripheral edema status.

Example 39: The system of any of examples 26-37 further comprising providing, via the second IMD, electrostimulation to the patient based on the peripheral edema status.

Example 40: The method of any of examples 21-37 further comprising providing, via an electrostimulation device, electrostimulation to the patient based on the peripheral edema status.

Example 41: A computer readable medium storing instructions that when executed by one or more processors cause the one or more processors to perform the method of any of examples 21-40.

Various examples have been described. These and other examples are within the scope of the following claims.

## Claims

1. A system comprising:
an implantable medical device (IMD) comprising:
a housing configured for subcutaneous implantation proximate a lymphatic element of a patient; and
a plurality of electrodes configured to sense an impedance signal indicative of one or more physiological parameters of the lymphatic element; and
processing circuitry configured to:
determine the one or more physiological parameters of the lymphatic element based on the impedance signal;
determine a peripheral edema status of the patient based on the one or more physiological parameters; and
output an indication of the peripheral edema status.

2. The system of claim 1, wherein the IMD is configured to be implanted in a limb of the patient.

3. The system of any of claims 1-2, wherein the impedance signal includes at least one of real impedance or reactive impedance.

4. The system of any of claims 1-3, wherein the physiological parameters include at least one of afferent lymph flow, efferent lymph flow, lymph node volume, lymph node geometric size, lymph node arterial waveform, lymph node arterial pressure, lymph node activity, inflammatory response, and/or cytokine release.

5. The system of any of claims 1-4, wherein the lymphatic element includes at least one of a lymph node or a lymph vessel of the patient.

6. The system of any of claims 1-5, wherein the IMD is a first IMD, the lymphatic element is a first lymphatic element, the physiological parameters are first physiological parameters of the first lymphatic element, the plurality of electrodes are a first plurality of electrodes, and the impedance signal is a first impedance signal, and
wherein the system further comprises a second IMD comprising:
a second housing configured for subcutaneous implantation proximate a second lymphatic element of the patient; and
a second plurality of electrodes configured to sense a second impedance signal indicative of one or more second physiological parameters of the second lymphatic element.

7. The system of claim 6, wherein the processing circuitry is further configured to:
determine one or more second physiological parameters based on the second impedance signal; and
determine the peripheral edema status based on the one or more first physiological parameters and the one or more second physiological parameters.

8. The system of any of claims 1-7 further comprising a computing device, wherein the computing device includes the processing circuitry.

9. The system of claim 8, wherein the computing device is separate from the first IMD and the second IMD, and
the processing circuitry is configured to receive the first impedance signal via communication circuitry of the first IMD and receive the second impedance signal via communication circuitry of the second IMD.

10. The system of any of claims 6-9, wherein the first IMD and the second IMD are each respectively configured to be implanted in a limb of the patient.

11. The system of any of claims 6-10, wherein the first impedance signal includes at least one of real impedance or reactive impedance and the second impedance signal includes at least one of real impedance or reactive impedance.

12. The system of any of claims 6-11, wherein the first lymphatic element includes at least one of a first lymph node or a first lymph vessel of the patient, and
the second lymphatic element includes at least one of a first lymph node or a second lymph vessel of the patient.

13. The system of any of claims 6-12, wherein the first IMD further comprises a first optical sensor configured to sense a first optical signal,
the second IMD further comprises a second optical sensor configured to sense a second optical signal, and
the processing circuitry is further configured to:
determine at least one of first lymph node arterial waveform or first lymph node arterial pressure based on the first optical signal;
determine at least one of second lymph node arterial waveform or second lymph node arterial pressure based on the second optical signal; and
determine the peripheral edema status based on the one or more first physiological parameters, the one or more second physiological parameters, the at least one of first lymph node arterial waveform or first lymph node arterial pressure, and the at least one of second lymph node arterial waveform or second lymph node arterial pressure.

14. The system of any of claims 1-13, wherein the IMD is configured to provide electrostimulation based on the peripheral edema status.

15. A computer readable medium storing instructions that when executed by one or more processors cause the one or more processors to perform:
sensing, via an implantable medical device (IMD) configured for subcutaneous implantation proximate a lymphatic element of a patient, an impedance signal indicative of one or more physiological parameters of the lymphatic element;
determining, via processing circuitry, the one or more physiological parameters of the lymphatic element based on the impedance signal;
determining, via the processing circuitry, a peripheral edema status of the patient based on the one or more physiological parameters; and
outputting, via the processing circuitry, an indication of the peripheral edema status.
